# EUROPÄISCHE PATENTSCHRIFT

(11) **EP 3 164 165 B1**
(45) Veröffentlichungstag und Bekanntmachung des Hinweises auf die Patenterteilung: **15.08.2018**
(21) Anmeldenummer: 15734342.7
(22) Anmeldetag: 30.06.2015
(51) Int. Cl.: A61L 27/16, A61L 27/48, A61L 27/50

(54) **AUTOPOLYMERISIERBARES PROTHESENMATERIAL SOWIE AUSPOLYMERISIERTER, BRUCHZÄHER PROTHESENWERKSTOFF MIT VERBESSERTER FARBSTABILITÄT**
AUTO-POLYMERIZABLE PROSTHETIC MATERIAL AND POLYMERIZED, FRACTURE-TOUGH PROSTHETIC MATERIAL WITH INCREASED COLOUR STABILITY
MATÉRIAU POUR PROTHÈSE AUTOPOLYMÉRISABLE ET MATÉRIAU PROTHÉTIQUE POLYMÉRISÉ RÉSISTANT AUX FÊLURES AVEC STABILITÉ DE COULEUR AMÉLIORÉE

(30) Priorität: 01.07.2014 DE 102014109234
(43) Veröffentlichungstag der Anmeldung: 10.05.2017
(73) Patentinhaber: Kulzer GmbH, 63450 Hanau (DE)
(72) Erfinder: RUPPERT, Klaus, 63477 Maintal (DE); HOHMANN, Alfred, 61389 Schmitten (DE); DEKERT, Stephan, 61273 Wehrheim (DE)
(74) Vertreter: Bendele, Tanja
(86) Internationale Anmeldenummer: PCT/EP2015/064866
(87) Internationale Veröffentlichungsnummer: WO 2016/001236

(56) Entgegenhaltungen:
- EP-A2- 1 702 633
- WO-A1-2015/017556
- DE-T2- 69 426 093

## Beschreibung

Gegenstand der Erfindung ist ein autopolymerisierbares 2-Komponenten-Prothesenbasismaterial und ein Verfahren zu seiner Herstellung umfassend (A) mindestens eine flüssige Monomerkomponente, und (B) mindestens eine pulverförmige Komponente, wobei das Prothesenmaterial in den Komponenten (A) und/oder (B), (i) mindestens einen Initiator oder ein Initiatorsystem für die Autopolymerisation, (ii) durch eine elastische Phase modifizierte Kern-Schale Partikel und (iii) mindestens ein Urethan(meth)acrylat, insbesondere ein Urethandimethacrylat, enthält.

Prothesenmaterialien neigen bei der Ausbettung aus dem Einbettmaterial (Gips) zum Abplatzen von dünn auslaufenden Bereichen. Ferner fallen Prothesen aufgrund der eingeschränkten Motorik der in der Regel älteren Prothesenträger beim Reinigen zuweilen auf einen harten Untergrund (Fliesen, Waschbecken), so dass es hier ebenfalls zu Abplatzern oder Brüchen kommen kann. Diese Abplatzer führen zu einer Zusatzarbeit und Zusatzkosten für das Dentallabor und sind daher unerwünscht. Ferner kann es insbesondere bei implantatgetragenem Zahnersatz aufgrund der deutlich höheren Kaukräfte zu Beschädigungen der Prothese kommen.

Aus diesen Gründen besteht die Anforderung nach einem Prothesenwerkstoff, welcher kurzzeitige Belastungen, wie die vorgenannten kurzzeitigen, hohen mechanischen Belastungen, ohne Materialschädigung toleriert. Diese Prothesenwerkstoffe werden als sogenannte High Impact-Werkstoffe bezeichnet. Die Anforderungen an diese Werkstoffe sind in der DIN ISO 20795-1 beschrieben. Produkte, welche diese Anforderungen erfüllen, sind schon seit einigen Jahren auf dem Markt, diese sind allerdings überwiegend der Gruppe der heisshärtenden Prothesenwerkstoffe zuzuordnen.

Als Kaltpolymerisate sind bisher nur zwei Produkte bekannt. Beide Produkte sind entweder sehr aufwendig zu verarbeiten (Ivobase High Impact/Fa. Ivoclar Vivadent: spezielles Verarbeitungsgerät, Spezialküvette, spezielle Einbettmasse, geschlossenes System) oder zeigen aufgrund der zugesetzten Additive ästhetisch nicht zufriedenstellende Farbveränderungen.

Aufgabe der Erfindung war die Bereitstellung eines Werkstoffes, insbesondere eines im medizinischen Bereich geeigneten Werkstoffes, bevorzugt eines Prothesenwerkstoffs, zur Kaltpolymerisation, der die Vorgaben der Norm DIN ISO 20795-1 hinsichtlich Bruchzähigkeit übertrifft. Ferner bestand die Aufgabe, dass der Werkstoff ohne aufwendige Zusatzgeräte herstellbar sein soll, insbesondere soll auch eine Verarbeitung ohne spezielle Zusatzgeräte möglich sein und nur mit typischen zahntechnischen Techniken und Geräten zu verarbeiten. Eine weitere Aufgabe bestand darin, einen hoch-bruchzähen Werkstoff, insbesondere einen Prothesenwerkstoff bereitzustellen, dessen Transparenz im Vergleich zum Grundwerkstoff nicht beeinträchtigt wird.

Es wurde überraschend gefunden, dass ein Werkstoff, insbesondere ein Prothesenwerkstoff/ -basismaterial, mit den geforderten Eigenschaften bezüglich der Bruchzähigkeit und vorzugsweise bezüglich der Vorgaben zur Transparenz, bereitgestellt werden kann, indem einem Prothesenbasismaterial Kern-Schale-Partikel und Urethan(meth)acrylate zugesetzt werden.

Grundsätzlich wurden die folgenden Technologien zum Erzielen einer hohen Bruchzähigkeit in Betracht gezogen, Kern-Schale Partikel oder spezielle elastifizierende Flüssigadditive wie z.B. Butadien-Copolymere, Butadien-Acrylnitril-Copolymere, Silikonacrylate oder Urethanacrylate. Die Kern-Schale-Partikel konnten bisher nicht für Kaltpolymerisate verwendet werden, da die gegenüber Heisspolymerisaten deutlich kürzere Anquellzeit nicht zur Anquellung der Kern-Schale Partikel ausreichend war.

Die genannten Flüssigadditive, wie z.B. Acrylnitril-Butadien-Copolymere, neigten zum Vergilben des Prothesenwerkstoffes. Dies geschieht typischerweise in Gegenwart von oxidierenden Substanzen wie z.B. Peroxiden oder Luftsauerstoff. Zudem weisen die Flüssigadditive, wie z.B. Silikonacrylate eine deutlich von PMMA unterschiedliche Brechzahl auf und führen dadurch typischerweise zur Ausbildung eines opaken Werkstoffs. Die Urethanacrylat-basierten Flüssigadditive, wie z.B. Urethanacrylate, führen oft zwar zu einer Verbesserung der Bruchzähigkeit von Materialien, wobei die Mindestanforderungen der ISO 20795-1 bezüglich der Bruchzähigkeit jedoch nicht erreicht werden können. Ein weiterer Nachteil einiger Additive zeigt sich in einer Wasseraufnahme während des Polymerisationsprozesses, dies führt zu einer unerwünschten Weissverfärbung des Prothesenwerkstoffes während der Tragezeit.

Erfindungsgemäss wird die Aufgabe durch die Verwendung von Kern-Schale-Partikeln und mindestens einem Urethanmethacrylat im Prothesenbasismaterial gelöst. Besonders gute Ergebnisse im auspolymerisierten Prothesenmaterial können durch eine Kombination von Kern-Schale Partikeln in der flüssigen Monomerkomponente in Kombination mit mindestens einem Urethanacrylat oder Urethanmethacrylat in der Flüssigkeit erzielt werden. Eine hohe Transparenz des Prothesenwerkstoffs konnte durch Auswahl spezifischer Kern-Schale Partikel mit einer Brechzahl ähnlich der des auspolymerisierten Prothesenwerkstoffes gewährleistet werden. Daher weisen die Kern-Schale Partikel vorzugsweise eine Brechzahl von etwa 1,49 auf (R.I. ∼ 1,4900).

Erfindungsgemäss besonders bevorzugte Kern-Schale-Partikel liegen aggregiert vor. Die Aufgaben können durch die Verwendung von aggregierten Kern-Schale Partikeln (unregelmässig geformte Aggregate, d₅₀ ∼ 50 - 300 µm) gelöst werden, die Primärteilchengrösse beträgt ca. 200 - 400 nm. Die Kern-Schale-Partikel liegen vermutlich aufgrund von Oberflächenwechselwirkungen im Feststoff aggregiert vor. Das Additiv wird mit der Flüssigkeit gemischt und bildet eine stabile Suspension, welche innerhalb von wenigen Wochen nur schwach sedimentiert. Durch die Suspendierung in MMA zerfallen die Aggregate relativ rasch in die Primärpartikel.

Durch die erfindungsgemässe Verwendung der Kern-Schale-Partikel als High-Impact Additiv, bekannt z.B. aus EP1702633 A2, in Kombination mit mindestens einem Urethanacrylat, vorzugsweise einem Urethanmethacrylat, lassen sich Prothesenwerkstoffe herstellen, welche die Anforderungen der ISO 20795-1 hinsichtlich High-Impact Eigenschaften erfüllen. Darüber hinaus kann ein Prothesenwerkstoff bereitgestellt werden, dessen Biegefestigkeit und E-Modul in der gleichen Grössenordnung wie Nicht-High-Impact Werkstoffe liegt und gleichzeitig hochtransparent und farbstabil ist. Die erfindungsgemässen Prothesen zeigen keine Weissverfärbungen durch Kontakt mit wasserhaltigen Materialien wie z.B. Dubliergele oder Gipse während und nach dem Polymerisationsprozess.

Ferner kann der erfindungsgemässe Prothesenwerkstoff mit üblichen zahntechnischen Methoden und Instrumenten gemischt und verarbeitet werden. Zur Verarbeitung können herkömmliche, dem Zahntechniker gebräuchliche Techniken und Hilfsmittel verwendet werden. Spezielle Einbettmassen, Gipse, Küvetten, Geräte etc. (wie z.B. beim Ivoclar-System) können vermieden werden.

Kern-Schale Partikel als High-Impact-Additiv in einem Dentalwerkstoff offenbart EP1702633A2.

Erfindungsgemäss wird die Aufgabe durch die Verwendung von Kern-Schale-Partikeln und mindestens einem Urethanmethacrylat im Prothesenbasismaterial gelöst. Besonders gute Ergebnisse im auspolymerisierten Prothesenmaterial können durch eine Kombination von Kern-Schale Partikeln in der flüssigen Monomerkomponente in Kombination mit mindestens einem Urethanacrylat oder Urethanmethacrylat in der Flüssigkeit erzielt werden. Eine hohe Transparenz des Prothesenwerkstoffs konnte durch Auswahl spezifischer Kern-Schale Partikel mit einer Brechzahl ähnlich der des auspolymerisierten Prothesenwerkstoffes gewährleistet werden. Daher weisen die Kern-Schale Partikel vorzugsweise eine Brechzahl von etwa 1,49 auf (R.I. ∼ 1,4900).

Erfindungsgemäss besonders bevorzugte Kern-Schale-Partikel liegen aggregiert vor. Die Aufgaben können durch die Verwendung von aggregierten Kern-Schale Partikeln (unregelmässig geformte Aggregate, d₅₀ ∼ 50 - 300 µm) gelöst werden, die Primärteilchengrösse beträgt ca. 200 - 400 nm. Die Kern-Schale-Partikel liegen vermutlich aufgrund von Oberflächenwechselwirkungen im Feststoff aggregiert vor. Das Additiv wird mit der Flüssigkeit gemischt und bildet eine stabile Suspension, welche innerhalb von wenigen Wochen nur schwach sedimentiert. Durch die Suspendierung in MMA zerfallen die Aggregate relativ rasch in die Primärpartikel.

Durch die erfindungsgemässe Verwendung der Kern-Schale-Partikel als High-Impact Additiv in Kombination mit mindestens einem Urethanacrylat, vorzugsweise einem Urethanmethacrylat, lassen sich Prothesenwerkstoffe herstellen, welche die Anforderungen der ISO 20795-1 hinsichtlich High-Impact Eigenschaften erfüllen. Darüber hinaus kann ein Prothesenwerkstoff bereitgestellt werden, dessen Biegefestigkeit und E-Modul in der gleichen Grössenordnung wie Nicht-High-Impact Werkstoffe liegt und gleichzeitig hochtransparent und farbstabil ist. Die erfindungsgemässen Prothesen zeigen keine Weissverfärbungen durch Kontakt mit wasserhaltigen Materialien wie z.B. Dubliergele oder Gipse während und nach dem Polymerisationsprozess.

Ferner kann der erfindungsgemässe Prothesenwerkstoff mit üblichen zahntechnischen Methoden und Instrumenten gemischt und verarbeitet werden. Zur Verarbeitung können herkömmliche, dem Zahntechniker gebräuchliche Techniken und Hilfsmittel verwendet werden.

Spezielle Einbettmassen, Gipse, Küvetten, Geräte etc. (wie z.B. beim Ivoclar-System) können vermieden werden.

Zur Abgrenzung von Prothesenmaterialien zu üblichen dentalen Materialien wird hervorgehoben, dass Prothesenmaterialien wesentliche Mengen an polymeren pulverförmigen Komponenten umfassen, wie PMMA (Poly(meth)methylacrylat) und/oder (Poly(ethyl)methacrylat, insbesondere zu grösser gleich 50 Gew.-% in der Gesamtzusammensetzung. Übliche Prothesenmaterialien werden in der Regel in einem Kit mit einer pulverförmigen Komponente und einer flüssigen Komponente angeboten. Dentale Materialien zur Herstellung von Füllungen basieren im Wesentlichen auf polymerisierbaren Monomeren die vorzugsweise mit einem Anteil von grösser 55 Gew.-% in den polymerisierbaren Zusammensetzungen vorliegen.

Erfindungsgemäss besonders gut verwendbar sind Kern-Schale Partikel, welche ggf. als Aggregat vorliegen, wobei diese Aggregate jedoch innerhalb der kurzen Anquellzeit in die einzelnen Partikel auftrennbar sind, insbesondere zerfallen sie nach Zugabe der flüssigen Monomerkomponente in die einzelnen Partikel.

Gegenstand der Erfindung ist ein autopolymerisierbares bzw. kaltpolymerisierbares 2-Komponenten-Prothesenbasismaterial, insbesondere ein polymerisierbares Prothesenmaterial, umfassend
A) mindestens eine flüssige Monomerkomponente,
B) mindestens eine pulverförmige Komponente,
wobei das Prothesenmaterial in Komponente (A) und/oder (B) (i) mindestens einen Initiator oder ein Initiatorsystem für die Autopolymerisation oder Kaltpolymerisation, (ii) durch eine elastische Phase modifizierte Kern-Schale Partikel und (iii) mindestens ein Urethan(meth)acrylat enthält, insbesondere ein Urethandimethacrylat, bevorzugt ein Bis(methacryloxy-2-ethoxycarbonyl-amino)-alkylen, Diurethanacrylat Oligomer, Alkyl-funktionelle Urethandimethacrylat Oligomere, Aromatisch-funktionalisierte Urethandimethacrylat Oligomere, aliphatische ungesättigte Urethanacrylate, Bis(methacryloxy-2-ethoxycarbonylamino) substituierter Polyether, aromatische Urethandiacrylat Oligomere, aliphatische Urethandiacrylat Oligomere, monofunktionelle Urethanacrylate, aliphatische Urethandiacrylate, hexafunktionelle aliphatische Urethanharze, aliphatisches Urethantriacrylat, UDMA, aliphatisches Urethanacrylat Oligomer, ungesättigte aliphatische Urethanacrylat.

Komponenten des Initiatorsystems können, wie nachfolgend erläutert, auf die Komponenten A) und B) aufgeteilt werden.

Das erfindungsgemässe Prothesenmaterial weist als auspolymerisiertes Prothesenmaterial vorzugsweise eine Bruchzähigkeit (kₘₐₓ; Höchstfaktor der Beanspruchungsintensität) von grösser gleich 1,9 MPa · m^{1/2}auf, insbesondere grösser gleich 2 MPa · m^{1/2}, und vorzugsweise zugleich eine Gesamtbrucharbeit (W_{f}) von grösser gleich 900 J/m². Besonders bevorzugt liegt die Bruchzähigkeit bei grösser gleich (≥) 2,1 MPa · m^{1/2}, vorzugsweise bei ≥ 2,3 MPa · m^{1/2}, ≥ 2,4 MPa · m^{1/2}. Weiter ist es bevorzugt, wenn die Brucharbeit zugleich grösser ≥ 900 J/m² ist, insbesondere grösser gleich ≥ 950 J/m², ≥ 1000 J/m², besonders bevorzugt grösser gleich 1030 J/m². Besonders bevorzugt ist zudem die Biegefestigkeit zudem grösser 65 MPa, besonders bevorzugt grösser 70 MPa, weiter bevorzugt grösser gleich 75 MPa. Ein besonders bevorzugtes Prothesenmaterial weist eine Bruchzähigkeit von > 2,3 MPa*m^{1/2} und eine Gesamtbrucharbeit von > 1000 J/m² auf.

Weiter ist es bevorzugt, wenn die Transparenz der unpigmentierten, auspolymerisierten Prothesen im Bereich von grösser gleich 85%, insbesondere grösser gleich 90% liegt. (gemessen an 3 mm dicken Platte)

Besonders bevorzugt umfasst die Komponente (A) mindestens eine flüssige Monomerkomponente, (i) mindestens einen Initiator oder ein Initiatorsystem für die Autopolymerisation oder einen Teil der Komponenten des Initiatorsystems, (ii) durch mindestens eine elastische Phase modifizierte Kern-Schale Partikel und (iii) mindestens ein Urethan(meth)acrylat, insbesondere Urethandimethacrylat.

Dabei ist es besonders bevorzugt, wenn die Komponente (ii) durch mindestens eine elastische Phase modifizierte Kern-Schale Partikel zu 0,001 bis 20 Gew.-% vorliegen, insbesondere bis 10 Gew.-%, bevorzugt bis 5 Gew.-%, in Bezug auf die Gesamtzusammensetzung der Komponente (A) und (iii) mindestens ein Urethan(meth)acrylat, vorzugsweise ein Urethandimethacrylat, von 0,001 bis 20 Gew.-%, vorzugsweise bis 10 Gew.-%, weiter bevorzugt bis 5 Gew.-%, in Bezug auf die Gesamtzusammensetzung der Komponente (A) vorliegt (d.h. auf 100 Gew.-% der Komponente (A)).

Das erfindungsgemässe Prothesenbasismaterial zeigt trotz des zugesetzten High Impact - Modifizierers (Kern-Schale Partikel und Urethan(meth)acrylat)s keine negative Beeinflussung des Suntests nach ISO 20795-1.

Die Kern-Schale Partikel werden auch als High-Impact Modifizierer bezeichnet.

Besonders bevorzugte Prothesenbasismaterialien weisen vorzugsweise Kern-Schale Partikel auf, in denen die Verteilung der elastischen Phase der modifizierten Kern-Schale Partikel ausgewählt ist aus den Möglichkeiten a bis d: a) elastischer Phase als Kern (z.B. aus Butylacrylat) in harter Aussenschale (z.B. aus PMMA) (Kern-Schale-Teilchen); b) mehrere elastische Phasen als Kerne in einer harten Matrix, c) Kern-Schale Teilchen aus a), in harter Matrix verteilt und d) harter Kern mit elastischer Phase als Aussenschale.
Erfindungsgemässe Kern-Schale Partikel könne ebenso den nachfolgenden multi-layer Aufbau aufweisen, e) einen innen liegenden Kern mit mehreren Schichten als Schalen und einer Aussenschale, wobei insbesondere (i) mindestens eine der Schalen, vorzugsweise die Aussenschale hart ist und die verbleibenden Schalen und der Kern jeweils unabhängig aus elastischen Phasen bestehen. In Alternativen können die elastischen Phasen und die harten Phasen auf die Schalen und den Kern anderweitig aufgeteilt sein.

Ferner weisen bevorzugte Kern-Schale Partikel eine Brechzahl ähnlich der des auspolymerisierten Prothesenwerkstoffes auf. Vorzugweise liegt die Brechzahl der Kern-Schale Partikel um 1,4900 mit einer Schwankungsbreite von plus/minus 0,02, insbesondere +/- 0,01. Erfindungsgemäss besonders bevorzugte Kern-Schale-Partikel liegen aggregiert vor. Dabei weisen die Aggregate der Kern-Schale Partikel, die regellos geformt sein können, als unregelmässig geformtes Aggregat einen mittleren Durchmesser d₅₀ ∼ 50 - 300 µm auf. Die bevorzugte Grösse der Primärteilchengrösse beträgt kleiner 500 nm, insbesondere bis 100 nm,, bevorzugt von 200 - 400 nm. Gleichfalls können Kern-Schale Partikel mit einer Primärpartikelgrösse von kleiner gleich 200 nm bis 2 nm, wie zwischen 150 bis 10 nm als Kern-Schale Partikel eingesetzt werden.

Vorzugsweise weisen die Kern-Schale Partikel einen Brechungsindex von 1,48 bis 1,60 auf, insbesondere von 1,49 bis 1,55. Besonders bevorzugt liegt der Brechungsindex der Kern-Schale Partikel im Bereich des Brechungsindexes von PMMA, PEMA, vorzugsweise liegt der Brechungsindex daher um 1, 48 bis 1,50.

Gleichfalls bevorzugt sind Kern-Schale Partikel deren Dichte bei 0,9 bis 1,5 g/ml, insbesondere von 0,95 bis 1,4 g/ml beträgt. Vorzugsweise liegt die Schüttdichte zugleich bei 0,1 bis 0,6 g/ml, bevorzugt bei 0,1 bis 0,6 g/ml.

Unter einer harten Aussenschale, harten Matrix, hartem Kern wird ein Material verstanden, das vorzugsweise eine geringere Elastizität als das Material der elastischen Phase aufweist. Vorzugsweise ist die Elastizität der harten Materialien mindestens 40% geringer als die der elastischen Phase. Bevorzugte anorganische harte Kerne zeigen unter dem Einfluss einer Kraft im Wesentlichen keine Verformung, während die organischen harten Materialien unter Einwirkung einer Kraft eine deutlich geringere Verformung erleiden als die elastische Phase. Die harten Materialien als harte Aussenschale, harte Matrix und/oder harter Kern stabilisieren die elastische Phase in ihrer Form. Eine elastische Phase wird aus mindestens einem elastischen Material gebildet, das unter der Einwirkung einer Kraft eine reversible Verformung erfährt. Die Verformung der elastischen Phase ist vorteilhaft ohne Krafteinwirkung vollständig reversibel.

Bevorzugte Komponenten B) umfassen vorzugsweise mindestens eine pulverförmige Komponente umfassend a) polymere Partikel umfassend Polymere in Form von Polymerpulver umfassend Polyalkyl(meth)acrylate, die optional vernetzt sind und als Homo- oder Co-Polymere vorliegen, wobei die Polymere auf mindestens einem der Monomere, umfassend eine (Meth-)acrylat-Gruppe basieren, ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylenglykolmono-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere, Polyamidpartikel, Polyamidfasern. Darüber hinaus können die polymeren Partikel auch Gemische dentaler Monomeren wie z.B. MMA und zusätzlich mindestens einen Vernetzer umfassen.

Besonders bevorzugt umfasst die pulverförmige Komponente Polymethylmethacrylat (PMMA) Perlen als polymere Partikel und/oder Splitterpolymerisate, insbesondere mit Teilchengrössen von 10 - 100 µm, und/oder basiert auf Co-Polymeren umfassend einpolymerisierte Comonomere Styrol, alpha-Methylstyrol, Vinyltoluol, substituierte Vinyltoluole wie Vinylbenzylchloride, Vinylhalogenide wie Vinylchlorid, Vinylester, wie Vinylacetat, heterocyclische Vinylverbindungen wie 2-Vinylpyridin, Vinylacetat und Vinylpropionat, Butadien, Isobutylen, 2-Chlorbutadien, 2-Methylbutadien, Vinylpyridin, Cyclopenten, (Meth)acrylsäureester, wie Methylmethacrylat, Ethylmethacrylat,Butylmethacrylat, Butylacrylat und Hydroxyethylmethacrylat, ferner Acrylnitril, Maleinsäure und Maleinsäurederivate, wie beispielsweise Maleinsäureanhydrid, Fumarsäure und Fumarsäurederivate wie Fumarsäureester, Acrylsäure, Methacrylsäure sowie Aryl(meth)acrylate wie Benzylmethacrylat oder Phenylmethacrylat sowie optional Mischungen dieser Comonomere und optional zusätzlich
b) anorganische Füllstoffe umfassend pyrogene oder Fällungskieselsäuren, Dentalgläser wie Aluminosilicatgläser oder Fluoroaluminosilicatgläser, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, insbesondere Mischoxide von SiO₂ und ZrO₂, Glasfasern und/oder Kohlenstofffasern sowie Mischungen umfassend die pulverförmigen Komponenten a) und b).

Die b) anorganischen Füllstoffe werden in der Regel zu 0 bis 10 Gew.-%, vorzugsweise von 0,0001 bis 3 Gew.-%, bezogen auf die gesamte Prothesenkunststoffzusammensetzung bzw. die Summe der Komponenten (A) und (B) eingesetzt. In der Komponente (B) in Bezug auf die Gesamtzusammensetzung der Komponente (B) von 100 Gew.-% liegen sie in der Regel im Bereich von 0 bis 20 Gew.-% vor, vorzugsweise 0,001 bis 10 Gew.-%.

Ebenfalls im Sinne der Erfindung sind polymere Partikel, die auf mindestens einem (Meth-)-acrylat Monomer mit nur einer (Meth-)acrylat-Gruppe basieren oder die auf der Mischung mindestens zweier dieser (Meth-)acrylat Monomere basiert.

Erfindungsgemässe Kern-Schale Partikel umfassen als elastische Phase vorzugsweise mindestens ein Poly-(n-butyl-acrylat) PBA, Butadien-Styrol-Copolymer, Nitril-Butadien-Copolymer, Silikonkautschuk -(Pfropfcopolymerisat), Polyurethanpolymerisat, Polyolefinbasiertes Polyurethan (Polybutadien-basiertes Polyurethan), das vorzugsweise in MMA vorliegen kann. Die Partikelgrösse der Kern-Schale Partikel kann kleiner gleich 500 nm, wie zwischen 50 nm bis 500 nm, insbesondere kleiner gleich 400 nm bis 100 nm, oder alternativ kleiner 100 nm bis 2 nm betragen, gleichfalls kann die elastische Phase auf Polydimethylsiloxan modifiziertem Polyurethanen und/oder Epoxy-funktionalisierten elastischen Phasen basieren.

Erfindungsgemässe Kern-Schale Partikel umfassen als harte Schale, harten Kern und/oder harte Matrix mindestens ein (Meth)acrylat Polymer, vorzugsweise ein Alkyl(meth)acrylatPolymer, wie PMMA; Polystyrol, einem Epoxy-funktionalisierten Kern, sowie Homo- oder Co-Kondensate der vorgenannten Polymere.

Besonders bevorzugte Kern-Schale Partikel verleihen dem auspolymerisierten Prothesenmaterial in Kombination mit dem Urethan(meth)acrylat High Impact Eigenschaften, mit einer Bruchzähigkeit von ≥ 1,9 MPa · m^{1/2}, vorzugsweise ≥ 2 MPa · m^{1/2} und eine Brucharbeit von ≥ 900 J/m². Bevorzugte Kern-Schale Partikel umfassen Aggregate mit d₅₀ < 400 µm und Primärpartikelgrössen von d₅₀ kleiner 500 nm. Weiter bevorzugt können die Primärpartikel der Kern-Schale Partikel grösser gleich 100 nm sein, insbesondere als d₅₀-Wert.

Gleichfalls geeignete Kern-Schale Partikel umfassen einen elastischen Kern umfassend Acrylat-Polymere mit harter Aussenschale, insbesondere mit einer Partikelgrösse kleiner 1 Micrometer. Weiter bevorzugt weisen die Kern-Schale-Partikel gegenüber polymerisierbaren Monomeren reaktive Gruppen auf, vorzugsweise ist die Aussenschale mit (Meth)acrylat-Gruppen funktionalisiert. Alternative Kern-Schale Partikel umfassen als festen Kern ein Siliciumdioxid und eine elastische Schale umfassend mindestens ein Nitril-Butadien-Copolymer. Weitere Kern-Schale Partikel können in einer Monomerflüssigkeit vorliegen.

Als Urethan(meth)acrylate eignen sich erfindungsgemäss vorzugsweise difunktionelle und mehrfachfunktionelle Urethan(meth)acrylate, wie insbesondere Urethandi(meth)acrylate, besonders bevorzugt ist das mindestens eine (iii) Urethandimethacrylat (UDMA) ausgewählt aus linearen oder verzweigten Alkyl-funktionalisierten Urethandimethacrylaten, Urethandimethacrylat funktionalisierten Polyethern, insbesondere bis(methacryloxy-2-ethoxycarbonylamino)-alkylen, Bis(methacryloxy-2-ethoxycarbonylamino) substituierter Polyether, vorzugsweise 1,6-bis(methacryloxy-2-ethoxycarbonylamino)-2,4,4-trimethylhexane. Geeignete Urethan(meth)acrylate sind unter den folgenden Markennamen erhältlich. Ebecryl 230 (aliphatisches Urethandiacrylat), Actilane 9290, Craynor 9200 (Di-Urethanacrylat Ooligomer), Ebecryl 210 (aromatische urethan-diacrylat Oligomere), Ebecryl 270 (aliphatische Urethandiacrylat Oligomer), Actilane 165, , Actilane 250, Genomer 1122 (monofunktionelles Urethan- acrylat), Photomer 6210 (cas no. 52404-33-8, aliphatisches Urethan-diacrylat), Photomer 6623 (hexafunctional aliphatic Urethan Resin), Photomer 6891 (aliphatisches urethan-triacrylat), UDMA, Roskydal LS 2258 (Aliphatisches Urethan-acrylat Oligomer), Roskydal XP 2513 (ungesättigtes aliphatisches Urethanacrylat).

Ferner ist Gegenstand der Erfindung ein Prothesenbasismaterial umfassend eine (A) flüssige Monomerkomponente, die mindestens ein Monomer, insbesondere eine Mischung von Monomeren umfasst von a) Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere, und/oder b) zwei- und/oder Mehrfachvernetzer (>2) umfassend 1,4-Butandiol-dimethacrylat (1,4-BDMA) oder Pentaerythritol-tetraacrylat, Bis-GMA-Monomer (Bisphenyl-A-Glycidyl-Methacrylat), Triethylen-glycoldimethacrylat (TEGDMA) und Diethylenglycoldimethacrylat (DEGMA), Tetraethylen-glykoldi(meth)acrylat, Decandioldi(meth)acrylat, Dodecandioldi(meth)acrylat, Hexyldecan-dioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere.

Als geeignete Alkylmethacrylate für die flüssige Komponente (A) sei auf Methyl-, Ethyl-, n-Propyl, i-Propyl-, n-Butyl, t-Butyl, i-Butyl, Benzyl- und Furfurylmethacrylate oder deren Mischungen verwiesen. Methylmethacrylat ist davon besonders bevorzugt.

Vorzugsweise umfasst die (A) flüssige Monomerkomponente
a.1) von > 85 Gew.-% mindestens eine monofunktionelle (Meth)acrylat-funktionelle Monomerkomponente, insbesondere 85 bis 90 Gew.-%,
a.2) von 0 - 15 Gew.-% mindestens eine difunktionelle Di(meth)acrylat-funktionelle Monomerkomponente, insbesondere 0,01 bis 15 Gew.-%, 1 bis 10 Gew.-%, und
a.3) von 0 - 10 Gew.-% mindestens ein (meth)acrylat-funktionellens trifunktionelles (Meth)acrylat oder ein mehrfachfunktionelles (Meth)acrylat auf, insbesondere 0,01 bis 10 Gew.-%, vorzugsweise 1,0 bis 8 Gew.-%,
b) von 0 - 5 Gew.-% Stabilisatoren und Aktivatoren, Initiatoren, insbesondere 0,01 bis 5 Gew.-%, vorzugsweise 0,1 bis 3 Gew.-%
c) von 0,001 bis 20 Gew.-% Urethan(meth)acrylat, insbesondere 0,01 bis 10 Gew.-%, bevorzugt von 0,5 bis 5 Gew.-%, besonders bevorzugt von 0,01 bis 3 Gew.-%,
d) von 0,001 bis 20 Gew.-% Kern-Schale Partikel, insbesondere 0,001 bis 10 Gew.-%, bevorzugt 1 bis 10 Gew.-%, besonders bevorzugt 0,001 bis 5 Gew.-%, vorzugsweise zwischen 0,5 bis 5 Gew.-%. wobei die Gesamtzusammensetzung der flüssigen Monomerkomponente A) 100 Gew.-% beträgt. Gleichfalls beträgt die Gesamtzusammensetzung aller Komponenten der pulverförmigen Komponente (B) 100 Gew.-%. Die Kern-Schale-Partikel werden in der Regel in den Monomeren suspendiert.

Erfindungsgemäss setzt sich die Gesamtzusammensetzung der pulverförmigen Komponente (B) wie folgt zusammen:
b.1) von 100 Gew.-% polymere Partikel und optional anorganische Füllstoffe oder ein Gemisch dieser, der Gehalt kann sich vorzugsweise wie folgt zusammensetzen von 100 bis 80 Gew.-% polymere Partikel, wie PMMA, PEMA, von 0 bis 20 Gew.-% anorganische Füllstoffe, wie Dentalgläser, Metalloxid- oder Mischoxidbasis (SiO₂, ZrO₂ und/oder TiO₂), insbesondere 0,01 bis 10 Gew.-%
b.2) von 0 - 5 Gew.-% zumindest einen Teil eines Initiatorsystems, insbesondere 0,01 bis 4 Gew.-%, und
b.3) von 0 - 5 Gew.-% Pigmente, Hilfsstoffe, Stabilisatoren oder Gemische umfassend mindestens eine der vorgenannten Komponenten, insbesondere 0,01 bis 4 Gew.-%, vorgenannte Gesamtzusammensetzung der pulverförmigen Komponente (B) beträgt 100 Gew.-%.

Bevorzugt können pulverförmige polymere Komponenten, wie PMMA, unterschiedlicher Partikelgrössen ggf. als Homo- und/oder Co-Polymere eingesetzt werden: a) Homo-Polymer Perle 1 (d₅₀ ∼ 40 - 50 µm) und b) Perle 2 (d₅₀ ∼ 55 - 70 µm), und optional Co-Polymer c) Perle 3 (d₅₀ ∼ 40 - 50 µm) eingesetzt werden.

Die Gesamtzusammensetzung (A) mit 100 Gew.-% und die Gesamtzusammensetzung (B) mit 100 Gew.-% werden dann im Gewichtsverhältnis von (A) zu (B) von 1 zu 20 bis 20 zu 1 gemischt, bevorzugt im Gewichtsverhältnis von (A) zu (B) von 5 zu 15 bis 9 zu 8, bevorzugt von 5 bis 8 zu 8 bis 12, vorzugsweise im Gewichtsverhältnis von (A) zu (B) von 7 zu 10, insbesondere mit einer Schwankungsbreite von plus/minus 1, vorzugsweise von 0,5.

Als weitere flüssige Monomere kommen die auf dem Dentalgebiet üblichen Monomere in Betracht: Beispiele sind radikalisch polymerisierbare monofunktionelle Monomere wie Mono(meth)-acrylate, Methyl-, Ethyl-, Butyl-, Benzyl-, Furfuryl- oder Phenyl(meth)acrylat,

Typische difunktionelle Monomere, auch als Vernetzer bzw. Mehrfachvernetzer bezeichnet, sind BDMA, 1,4-Butandiol-dimethacrylat (1,4-BDMA), Bis-GMA-Monomer (Bisphenyl-A-Glycidyl-Methacrylat, (ein Additionsprodukt aus Methacrylsäure und Bisphenol-A-diglycidyl-ether), Diethylenglykoldi(meth)acrylat, Bisphenol-A-di(meth)acrylat, Decandioldi(meth)acrylat, Dodecandioldi(meth)acrylat, Hexyldecandioldi(meth)acrylat, sowie Butandioldi(meth)acrylat, Ethylenglycol-di(meth)acrylat, Polyethylenglycol-di(meth)acrylate, ethoxylierte/propoxylierte Bisphenol-A-di(meth)acrylate. Die folgenden difunktionellen Monomere können auch als Verdünnungsmittel (dünnflüssige Acrylate wie Triethylenglycoldimethacrylat (TEGDMA) und Diethylenglycoldimethacrylat (DEGMA), zugesetzt werden. Tri- und tetraafunktionelle Monomere bzw. Mehrfachvernetzer umfassen Tri- oder Tetraethylenglykoldi(meth)acrylat, Trimethylolpropan-tri(meth)acrylat, tris(2-Hydroxyethyl)-isocyanurat-triacrylat, Pentaerythritol-tetraacrylat, Weitere Vernetzer sind nachfolgend auch unter den polymeren Partikeln umfassend Co-Polymere offenbart, die mindesten ein (Meth-)acrylat Monomer mit zwei, drei, vier, fünf oder sechs (Meth-)acrylat-Gruppen umfassen.

Der Anteil an bei Raumtemperatur flüssigem aliphatischem (Meth)acrylat im erfindungsgemässen, gemischten, noch nicht auspolymerisiertem Prothesenbasismaterial, insbesondere umfassend die Komponenenten (A) und (B), beträgt beispielsweise 0,5 % bis 40 Gew.%, bevorzugt 20 bis 40 Gew.% Das aliphatische (Meth)acrylat kann in der flüssigen Monomerkomponente (A) oder optional zumindest teilweise in der Feststoffkomponente bzw. Pulverkomponente (B) oder in beiden zugegen sein. Bevorzugt befindet es sich in Komponente (A).

Ferner ist Gegenstand der Erfindung ein Prothesenbasismaterial, das vorzugsweise in Komponente (A), (B) oder in (A) und (B) zusätzlich mindestens einen oder mehrere Stoff(e) aus den Gruppen der Füllstoffe, Pigmente, Stabilisatoren, Regler, antimikrobiellen Additive, UV-Absorber, Thixotropiermittel, Katalysatoren und Vernetzer enthält. Solche Additive werden - wie auch Pigmente, Stabilisatoren und Regler - in eher geringen Mengen eingesetzt, z. B. insgesamt zu 0,01 bis 3,0, besonders 0,01 bis 1,0 Gew.% bezogen auf die Gesamtmasse des Materials. Geeignete Stabilisatoren sind z. B. Hydrochinonmonomethylether oder 2,6-Di-tert.-butyl-4-methylphenol (BHT).

Gleichfalls Gegenstand der Erfindung ist ein Prothesenbasismaterial das optional zusätzlich mindestens einen Initiator oder mindestens ein Initiatorsystem für die Autopolymerisation aufweist, das je nach Reaktionsbedingungen bzw. Polymerisationssystem, in der flüssigen Komponente (A), der pulverförmigen Komponente (B) oder in (A) und (B) vorliegen.

Die folgenden Initiatoren und/oder Initiatorsysteme für die Auto- oder Kaltpolymerisation umfassen a) mindestens einen Initiator insbesondere mindestens ein Peroxid und/oder Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid, und optional b) mindestens einen Aktivator, insbesondere mindestens ein aromatisches Amin, wie N,N-Dimethyl-p-toluidin, N,N-Dihydroxyethyl-p-toluidin und/oder p-Dibenzylaminobenzoesäurediethylester oder c) mindestens ein Initiatorsystem ausgewählt aus Redoxsystemen, insbesondere eine Kombinationen ausgewählt aus Dibenzoylperoxid, Dilauroylperoxid und Campherchinon mit Aminen ausgewählt aus N,N-Dimethyl-p-toluidin, N-N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend (i) Barbitursäure oder Thiobarbitursäure oder ein Barbitursäure- oder Thiobarbitursäurederivat und (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat und Benzyldibutylammoniumchlorid. Besonders bevorzugt wird die Polymerisation im 2-Komponenten Prothesenbasismaterial über ein Barbitursäurederivat gestartet.

Gegenstand der Erfindung ist auch ein Verfahren zur Herstellung eines auspolymerisierten Prothesenbasismaterial, sowie ein Prothesenmaterial erhältlich nach dem Verfahren, indem die Komponenten A) mindestens eine flüssige Monomerkomponente, und B) mindestens eine pulverförmige Komponente gemischt werden und nachfolgend auspolymerisiert bzw. gehärtet werden.

Besonders bevorzugt umfasst A) die flüssige Monomerkomponente Methylmethacrylat, Butandiolmethacrylat und optional mindestens ein methacrylat-basiertes di-, tri- und oder tetrafunktionelles Monomer als Mehrfachvernetzer, wie beispielsweise tris(2-Hydroxyethyl)-isocyanurat-triacrylat und/oder Pentaerythritol-tetraacrylat, sowie ein Urethandiacrylat, zumindest ein Teil eines Initiatorsystems und B) umfasst PMMA Perlen, Barbitursäure oder ein Barbitursäurederivat, sowie optional Pigmente. Zur Herstellung des Prothesenbasismaterials werden die Komponenten A) und B) gemischt.

Nach einer bevorzugten Verfahrensvariante werden die A) Monomerkomponente und die B) pulverförmige Komponente im Gewichtsverhältnis von 1 : 50 bis 50 : 1, insbesondere im Gewichtsverhältnis 7 : 10 (10 g Pulver, 7 ml Flüssigkeit, mit ähnlicher Dichte) gemischt, insbesondere mit einer Schwankungsbreite von plus/minus 1, bevorzugt plus/Minus 0,5.

Das Mischen der Komponenten A) und B) kann erfindungsgemäss mittels einfacher dem Zahntechniker bekannter Massnahmen erfolgen, wie mittels Spatel.

Ebenso ist Gegenstand der Erfindung ein pigmentiertes, auspolymerisiertes Prothesenbasismaterial. Ferner ist Gegenstand der Erfindung ein unpigmentiertes, auspolymerisiertes Prothesenbasismaterial mit einer Transparenz von grösser 85 % (gemessen an 3 mm Platten).

Nach einer weiteren Ausgestaltung ist Gegenstand der Erfindung die Verwendung von mit mindestens einer elastischen Phase modifizierten Kern-Schale Partikeln und mindestens einem Urethandi(meth)acrylat oder einem Derivat eines Urethandi(meth)acrylats in auto- oder Kaltpolymerisierbaren Prothesenmaterialien.

Ebenso Gegenstand der Erfindung ist die Verwendung des Prothesenmaterials im humanen Dentalbereich, insbesondere für Dentalprothesen, Teile von Prothesen, zur Anfertigung von Aufbissschienen, von Bohrschablonen für die Implantologie, von Mouthguards, als Knochenzement, als Knochenzement zur Zementierung von künstlichen Gelenkprothesen, Kronen, Teleskope, Veneers, Zahnbrücken, Prothesenzähne, Implantate, Implantateile, Abutments, Suprastrukturen, Kieferorthopädische Apparate und Instrumente, im Veterinärbereich, insbesondere für Hufreparaturmaterialien, als Knochenzement, als Knochenzement zur Zementierung von künstlichen Gelenkprothesen, Kieferorthopädische Apparate und Instrumente.

Nach einer weiteren Alternative ist Gegenstand der Erfindung ein Kit umfassend ein autopolymerisierbares Prothesenmaterial, wobei das Kit separierte Komponenten (A) und (B) umfasst.

Als auto- oder kaltpolymerisierbar gilt ein Prothesenmaterial gemäss der Erfindung, wenn die Kriterien nach ISO 20795-1 (Pkt. 3.1) erfüllt sind. Als kaltpolymerisierende Kunststoffe gelten Zusammensetzungen, die unterhalb 65 °C polymerisieren. Erfindungsgemässe kaltpolymerisierende Prothesenmaterialien können vorzugsweise in einem Temperaturbereich nach Mischen der beiden Komponenten (A) und (B) von 50 °C bis 65 °C, vorzugsweise von 50 bis 60 °C, weiter bevorzugt von 50 °C bis 55 °C selbständig aushärtet bzw. auspolymerisieren. Entsprechend der vorstehenden Norm werden polymerisierbare Zusammensetzungen, die ab 65 °C selbständig aushärten bzw. auspolymerisieren, als heisshärtende Zusammensetzungen, bezeichnet.

Die Pulverkomponente des Zweikomponenten-Prothesenbasismaterials enthält in der Regel ein Polymerpulver, insbesondere auf Methacrylatbasis, und/oder ein Perlpolymerisat auf Methacrylat-Basis. Perlpolymerisate werden auf diesem Gebiet häufig als Pulver bezeichnet.

Perlpolymerisate, insbesondere solche aus (Meth)acrylaten, sind dem Fachmann bekannt. Perlpolymerisate auf der Basis von Polyalkyl(meth)acrylaten erhält man in bekannter Weise durch Fällungspolymerisation oder Suspensionspolymerisation. Dabei liefert die Suspensionspolymerisation in der Regel grössere Teilchen. Die durchschnittlichen Teilchengrössen decken eine weite Spanne ab und können beispielsweise von 0,1 µ bis 250 µm betragen. Es kann sich auch um vernetzte Perlpolymerisate handeln. Geeignete multifunktionelle Vernetzermoleküle sind der obigen Aufzählung der auf dem Dentalgebiet üblichen Monomeren zu entnehmen.

In das Perlpolymerisat können auch weitere Comonomere, wie vorstehend erläutert, einpolymerisiert sein.

In einer bevorzugten Ausgestaltung sind in die Perlen des ersten (Co)polymerisats und/oder in die Perlen des zweiten (Co)polymerisats zumindest teilweise Vernetzer einpolymerisiert worden. Die ersten und zweiten Perlpolymerisate umfassen somit auch vernetzte und teilvernetzte Perlpolymerisate.

Für die Vernetzung greift man regelmässig auf multifunktionelle Comonomere oder auch multifunktionelle Oligomere zurück. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, ausserdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben.

Der Gehalt an derartigen Vernetzermolekülen liegt vorzugsweise im Bereich von 0,1 Gew.-% bis 10 Gew.-%, insbesondere im Bereich von 0,5 Gew.-% bis 5 Gew.-%, in der Ausgangsmischung für das Perlpoymerisat.

In einer zweckmässigen Ausgestaltung liegt in der flüssigen Komponente (A) neben mindestens einem der vorangehend genannten monofunktionellen Alkylmethacrylatmonomere mindestens ein Vernetzer vor. Dabei handelt es sich beispielsweise um multifunktionelle Monomere, Comonomere oder auch multifunktionelle Oligomere. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, ausserdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben. Selbstverständlich können auch Mischungen der genannten Vernetzermoleküle zum Einsatz kommen. Besonders geeignet sind auch solche multifunktionellen Verbindungen, insbesondere di- und/oder tri-funktionelle Verbindungen, die elastische Einheiten besitzen und demgemäss geeignet sind, die aus den Prothesenausgangsmaterialien erhaltenen Prothesenmaterialien mit flexiblen Eigenschaften auszustatten.

Exemplarisch sei auf die Dimethacrylate, wie 1,4-Butandiol-dimethacrylat, verwiesen. Solche Vernetzermoleküle können in der flüssigen Monomerkomponente (A) in Mengen im Bereich von 0,1 bis 20 Gew.-%, vorzugsweise im Bereich von 1 bis 10 Gew.-%, beispielsweise 5 Gew.-% zugegen sein.

In einer weiteren Ausführungsform können in der Flüssigkomponente (A) neben z. B. Methylmethacrylat als bevorzugtem Hauptmonomer (> 50 Gew.-%) weitere Comonomere vorliegen.

Das für die Polymerisation erforderliche radikalische Initiatorsystem ist, je nach Reaktionsbedingungen bzw. Polymerisationssystem, in der flüssigen Komponente (A) und/oder der pulverförmigen Komponente (B) enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der pulverförmigen Komponente vor und wird demgemäss beim Mischen dieser Komponenten zusammengeführt. Folglich liegt in der Regel eine Initiatorkomponente (c) in der pulverförmigen Komponente (B) vor, insbesondere in Form von Peroxiden, Perketalen, Perestern und/oder Azoverbindungen. Ein weiterer Teil des Initiatorsystems (c) kann sich in der Flüssigkomponente (A) befinden, in der Regel ein Coinitiator. Als Initiatoren können auch Restgehalte an bei der Herstellung der pulverförmigen Komponenten nicht abreagierter Initiatorkomponente verwandt werden, z. B. Peroxide wie Dibenzoylperoxid.

Als Initiatoren für die Polymerisationsreaktion von kalt- bzw. autopolymerisierenden Ausgangsmischungen kommen grundsätzlich solche in Betracht, mit denen sich radikalische Polymerisationsreaktionen starten lassen. Bevorzugte Initiatoren sind Peroxide sowie Azoverbindungen, wie zum Beispiel die folgenden: LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid.

Um die Initiierung der radikalischen Polymerisation durch Peroxide zu beschleunigen, können geeignete Aktivatoren, z. B. aromatische Amine, hinzugefügt werden. Exemplarisch seien als geeignete Amine N,N-Dimethyl-p-toluidin, N,N-Dihydroxyethyl-p-toluidin und p-Dibenzylaminobenzoesäurediethylester genannt. Hierbei fungieren die Amine regelmässig als Co-Initiatoren und liegen üblicherweise in einer Menge von bis zu 0,5 Gew.-% vor.

Als radikalische Initiatorsysteme sind die genannten Redoxsysteme geeignet. In einer zweckmässigen Ausführungsform enthält ein solches Redoxsystem Barbitursäure oder Thiobarbitursäure oder ein Barbitursäure- oder Thiobarbitursäurederivat (beispielsweise 25 bis 80 Gew.-%), mindestens ein Kupfersalz oder ein Kupferkomplex (beispielsweise 0,1 bis 8 Gew.-%) und mindestens eine Verbindung mit einem ionogen vorliegenden Halogenatom (beispielsweise 0,05 bis 7 Gew.-%). Exemplarisch seien als geeignete Bestandteile des vorangehend genannten Redoxsystems 1-Benzyl-5-Phenylbarbitursäure, Kupferacetylacetonat und Benzyldibutylammoniumchlorid genannt.

Die Aushärtung der Zusammensetzungen erfolgt vorzugsweise durch redoxinduzierte radikalische Polymerisation bei Raumtemperatur bzw. bei leicht erhöhter Temperatur unter leichtem Druck, um Blasenbildung zu vermeiden. Als Initiatoren für die bei Raumtemperatur durchgeführte Polymerisation werden z.B. Redoxinitiator-Kombinationen, wie z.B. Kombinationen von Benzoyl- oder Laurylperoxid mit N,N-Dimethyl-sym.-xylidin oder N,N-Dimethyl-p-toluidin, verwendet. Ein besonders bevorzugtes Initiatorsystem ist eine Kombination von Barbitursäuren in Verbindung mit Kupfer- und Chlorid-ionen sowie oben genannten Peroxiden. Dieses System zeichnet sich durch eine hohe Farbstabilität aus.

Ferner kann man die pulverförmige Komponente (B) und/oder die Flüssigkomponente (A) in bekannter Weise mit weiteren Zusatzstoffen aus den Gruppen der Stabilisatoren, UV-Absorbern, Thixotropiermitteln und Füllstoffen versehen.

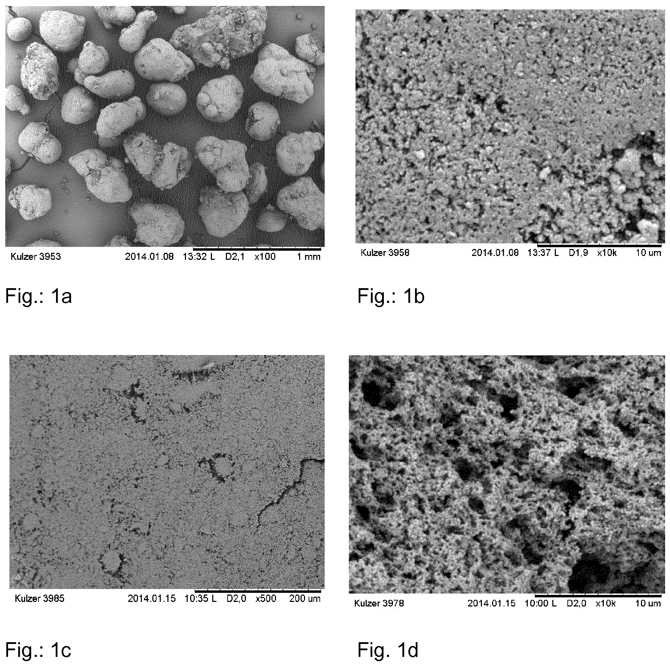

Die Figuren 1a bis d beschreiben den Erfindungsgegenstand näher, ohne ihn auf diese Ausführungsformen zu beschränken.
Figur 1a und Figur 1b: REM - Aufnahmen der Kern-Schale Partikel (links: 100x, rechts 10.000x)
Figur 1c und d. REM - Aufnahme des in MMA suspendierten und vor der Aufnahme getrockneten Additivs (links: 500x, rechts 10.000x)

### Ausführungsbeispiel:

Herstellen der erfindungsgemässen Pulvermischung (B): Aus PMMA-Perlen unterschiedlicher Korngrösse (Perle 1 Homo-Polymerisat (d50 ∼ 40 - 50 µm) 60 - 70 %, Perle 2 (d50 ∼ 55 - 70 µm) 15 %, Perle 3 Co-Polymerisat (d50 ∼ 40 - 50 µm) 15 %) wird unter Zusatz von Barbitursäure und Farbpigmenten eine Mischung hergestellt.

### Herstellen der erfindungsgemässen Flüssigkeit/Monomermischung (A):

Aus Methylmethacrylat, und einem weiteren methacrylat-basierten Mehrfach-Vernetzer wird unter Zusatz von Stabilisatoren und Initiatoren (Barbitursäure / Kupfer - System) eine Mischung hergestellt. Ferner wird das erfindungsgemässe Urethandimethacrylat sowie der erfindungsgemässe Kern-Schale-Partikel zugesetzt.

Erfindungsgemässes Beispiel Pulver- und Flüssigkeitmischung:

| **Flüssigkeit** | Beispiel 1 | VG 1 ohne Kern-Schale und ohne Urethan(meth-) acrylat | VG2 ohne Kern-Schale und mit Urethan(meth-) acrylat | VG3 mit Kern-Schale und ohne Urethan(meth-) acrylat |
|---|---|---|---|---|
| Methylmethacrylat | 93,3 | 98,3 | 95,3 | 96,3 |
| 2-(4,6-diphenyl-1,3,5-triazin-2-yl)-5-((hexyl)oxy)-phenol | 0,3 | 0,3 | 0,3 | 0,3 |
| N-Methyl-N,N-dioctyloctan-1-ammoniumchlorid | 0,2 | 0,2 | 0,2 | 0,2 |
| Kupfer(II) Chlorid Lsg. | 0,1 | 0,1 | 0,1 | 0,1 |
| NN-Bis(2-hydroxyethyl)-p-toluidin | 0,1 | 0,1 | 0,1 | 0,1 |
| ges. | 94,00 | 99,00 | 96,00 | 97,00 |
| | | | | |
| difunktionales aliphatisches urethanacrylat oligomer | 3,00 | - | 3,00 | - |
| tris(2-hydroxy ethyl) isocyanurat-triacrylate | 1,00 | 1,00 | 1,00 | 1,00 |
| Kern-Schale Partikel | 2,00 | - | - | 2,00 |
| ges. | 100,00 | 100,00 | 100,00 | 100,00 |

| **Pulver** | | | | |
|---|---|---|---|---|
| PMMA/PMA d₅₀ ∼ 40-45 µm | 67,5 | 67,5 | 67,5 | 67,5 |
| PMMA d₅₀ ∼ 40-55 µm | 15,000 | 15,000 | 15,000 | 15,000 |
| Vernetztes PMMA d₅₀ ∼ 55-70 µm | 15,000 | 15,000 | 15,000 | 15,000 |
| Phenylbenzylbarbitursäure | 2,5 | 2,5 | 2,5 | 2,5 |
| ges. | 100,000 | 100,000 | 100,000 | 100,000 |

**Tabelle 1: Gegenüberstellung des erfindungsgemässen Beispiels und der Vergleichsbeispiele**

| Physikalische Eigenschaften (Norm ISO 20795-1) | | | | | |
|---|---|---|---|---|---|
| | | Besp. 1 | VG1 | VG2 | VG3 |
| Biegefestigkeit [MPa] | >60 | 69,7 | 69,1 | 67,8 | 70,5 |
| E-Modul [MPa] | >1500 | 2365 | 2417 | 2374 | 2397 |
| Bruchzähigkeit [MPa m1/2] | >1,9 | 2,45 | 1,77 | 1,77 | 2,39 |
| Gesamtbrucharbeit [J/m²] | >900 | 1041,18 | 325,61 | 340,37 | 856,67 |
| Transparenz [delta %] (nach 6 d/ 37 °C in Ringerlösung) | | -3,16 | -7,04 | -4,88 | -3,36 |

Herstellung von Prüfkörpern, Bestimmung der Farbwerte, Bestimmung der mechanischen Eigenschaften:
**Farbprüfkörper:** Folgende Pulver- und Monomermischungen werden im Verhältnis 10 g Pulver zu: 7 ml Flüssigkeit intensiv gemischt und nach der Anquellphase (ca. 5 min bei 23 °C) Prüfkörper mit einer Abmessung von 30 x 30 x 3 mm gegossen und 30 min. bei 55°C und 2 bar Druck im Palamat elite auspolymerisiert.
**Prüfkörper für mechanische Festigkeit:** Folgende Pulver- und Monomermischungen werden im Verhältnis 10 g Pulver: 7 ml Flüssigkeit intensiv gemischt und nach der Anquellphase (ca. 5 min bei 23 °C) Prüfkörper mit einer Abmessung von 100 x 100 x 5 mm gegossen und 30 min. bei 55°C und 2 bar Druck im Palamat elite auspolymerisiert.

Die Prüfplatten werden anschliessend auf die in der ISO 20795-1 angegebene Geometrie zurecht gesägt und geschliffen.

Prüfkörper für mechanische Abprüfungen werden in Stahlformen, Prüfkörper für farbmetrische Abprüfungen werden in Dubliergel hergestellt.

Zur Bestimmung der Transparenz werden die Prüfkörper für 5 Tage bei 37°C in Ringer-Lösung eingelagert und die Farbwerte und Transparenz an 3 mm-Platten jeweils vor und nach der Einlagerung bestimmt. Die Messungen der Tabelle 1 wurden gemäss der DIN EN ISO 20795-1 durchgeführt.

Die erfindungsgemässe Monomermischung zeigt eine deutlich verbesserte Bruchzähigkeit gegenüber allen Vergleichsbeispielen, eine erhöhte Transparenz sowie den geringsten Transparenzverlust nach einer Lagerung in einer Ringerlösung (reduzierte Neigung zu Weissverfärbung).

## Patentansprüche

1. Autopolymerisierbares 2-Komponenten-Prothesenbasismaterial umfassend
A) mindestens eine flüssige Monomerkomponente,
B) mindestens eine pulverförmige Komponente,
**dadurch gekennzeichnet, dass**
das Prothesenmaterial in Komponente (A)
(i) mindestens einen Initiator oder ein Initiatorsystem für die Autopolymerisation,
(ii) durch eine elastische Phase modifizierte Kern-Schale Partikel und
(iii) mindestens ein Urethandimethacrylat enthält.

2. Prothesenbasismaterial nach Anspruch 1, **dadurch gekennzeichnet, dass** die Verteilung der elastischen Phase der modifizierten Kern-Schale Partikel ausgewählt ist aus den Möglichkeiten a bis d
a) elastischer Phase als Kern in harter Aussenschale (Kern-Schale-Teilchen);
b) mehrere elastische Phasen als Kerne in einer harten Matrix,
c) Kern-Schale Teilchen aus a), in harter Matrix verteilt und
d) harter Kern mit elastischer Phase als Aussenschale.

3. Prothesenmaterial nach Anspruch 1 oder 2, **dadurch gekennzeichnet, dass** die pulverförmige Komponente umfasst
a) polymere Partikel umfassend Polymere in Form von Polymerpulver umfassend Polyalkyl(meth)acrylate, die optional vernetzt sind und als Homo- oder Co-Polymere vorliegen, wobei die Polymere auf mindestens einem der Monomere, umfassend eine (Meth-)acrylat-Gruppe basieren, ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylen-glykol-mono-acrylat,Tetrahydrofuryl-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere, Polyamidpartikel, Polyamidfasern,
und optional
b) anorganische Füllstoffe umfassend pyrogene oder Fällungskieselsäuren, Dentalgläser wie Aluminosilicatgläser oder Fluoroaluminosilicatgläser, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, insbesondere Mischoxide von SiO₂ und ZrO₂, Glasfasern und/oder Kohlenstofffasern sowie definierte Mischungen umfassend die pulverförmigen Komponenten a) und b).

4. Prothesenmaterial nach einem der Ansprüche 1 bis 3, **dadurch gekennzeichnet, dass** die pulverförmige Komponente Polymethylmethacrylat (PMMA) Perlen als polymere Partikel und/oder Splitterpolymerisate, Co-Polymere enthält, umfassend als einpolymerisierte Comonomere Styrol, alpha-Methylstyrol, Vinyltoluol, substituierte Vinyltoluole wie Vinylbenzylchloride, Vinylhalogenide wie Vinylchlorid, Vinylester, wie Vinylacetat, heterocyclische Vinylverbindungen wie 2-Vinylpyridin, Vinylacetat und Vinylpropionat, Butadien, Isobutylen, 2-Chlorbutadien, 2-Methylbutadien, Vinylpyridin, Cyclopenten, (Meth)acrylsäureester, wie Methylmethacrylat, Butylmethacrylat, Butylacrylat und Hydroxyethylmethacrylat, ferner Acrylnitril, Maleinsäure und Maleinsäurederivate, wie beispielsweise Maleinsäureanhydrid, Fumarsäure und Fumarsäurederivate, wie Fumarsäureester, Acrylsäure, Methacrylsäure, Aryl(meth)acrylate, wie Benzylmethacrylat oder Phenylmethacrylat sowie optional Mischungen dieser Comonomere

5. Prothesenmaterial nach einem der Ansprüche 1 bis 4, **dadurch gekennzeichnet, dass** die elastische Phase ausgewählt ist aus Poly-(n-butyl-acrylaten) (PBA), Butadien-Styrol-Copolymeren, Nitril-Butadien-Copolymere, Silikonkautschuk-(Pfropfcopolymerisaten), Polyurethanpolymerisaten, Polyolefin-basierten Polyurethanen (Polybutadien-basierte Polyurethanen; Polydimethylsiloxan modifizierten Polyurethanen, Epoxy-funktionalisierten elastischen Phasen.

6. Prothesenmaterial nach einem der Ansprüche 1 bis 5, **dadurch gekennzeichnet, dass** die harte Schale, der harte Kern und/oder die harte Matrix ausgewählt sind aus (Meth)acrylat Polymeren, vorzugsweise Alkyl(Meth)acrylat-Polymeren, wie PMMA; Polystyrol, Epoxyfunktionalisierte Schale, Metalloxid Partikel kleiner 100 nm als Kern, wie Siliciumdioxid, Zirkondioxid oder Siliciumdioxid und Zirkondioxid enthaltende Partikel, sowie Homo- oder Co-Kondensate der vorgenannten Polymere.

7. Prothesenbasismaterial nach einem der Ansprüche 1 bis 6, **dadurch gekennzeichnet, dass** das (iii) Urethandimethacrylat vorzugsweise ausgewählt ist aus linearen oder verzweigten Alkyl-funktionalisierten Urethandimethacrylaten, Urethandimethacrylat funktionalisierten Polyethern, insbesondere bis(methacryloxy-2-ethoxycarbonylamino)-alkylen, bis(methacryloxy-2-ethoxycarbonylamino) substituierter Polyether, vorzugsweise 1,6-bis(methacryloxy-2-ethoxycarbonylamino)-2,4,4-trimethylhexane.

8. Prothesenbasismaterial nach einem der Ansprüche 1 bis 7, **dadurch gekennzeichnet, dass** die flüssige Monomerkomponente mindestens ein Monomer oder eine Mischung von Monomeren umfasst von
a) Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere, und/oder
b) zwei- und/oder Mehrfachvernetzer umfassend 1,4-Butandiol-dimethacrylat (1,4-BDMA) oder Pentaerythritol-tetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere.

9. Prothesenbasismaterial nach einem der Ansprüche 1 bis 8, **dadurch gekennzeichnet, dass** die Primärpartikel der Kern-Schale Partikel von 500 nm bis 10 nm betragen.

10. Prothesenbasismaterial nach einem der Ansprüche 1 bis 9, **dadurch gekennzeichnet, dass** es zusätzlich mindestens einen Initiator oder mindestens ein Initiatorsystem für die Autopolymerisation aufweist, die je nach Reaktionsbedingungen bzw. Polymerisationssystem, in der flüssigen Komponente (A), der pulverförmigen Komponente (B) oder in (A) und (B) vorliegen.

11. Prothesenbasismaterial nach einem der Ansprüche 1 bis 10, **dadurch gekennzeichnet, dass** der mindestens eine Initiator oder das mindestens eine Initiatorsystem für die Autopolymerisation umfasst
a) mindestens einen Initiator insbesondere mindestens ein Peroxid, Perketal, Perester und/oder eine Azoverbindung, und optional
b) mindestens einen Aktivator, insbesondere mindestens ein aromatisches Amin, oder
c) mindestens ein Initiatorsystem ausgewählt aus Redoxsystemen, insbesondere eine Kombinationen ausgewählt aus Dibenzoylperoxid, Dilauroyl und Campherchinon mit Aminen ausgewählt aus N,N-Dimethyl-p-toluidin, N-N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend (i) Barbitursäure oder Thiobarbitursäure oder ein Barbitursäure- oder Thiobarbitursäurederivat und (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat und Benzyldibutylammoniumchlorid.

12. Prothesenbasismaterial nach einem der Ansprüche 1 bis 11, umfassend (ii) durch mindestens eine elastische Phase modifizierte Kern-Schale Partikel von 0,001 bis 20 Gew.-% in Bezug auf die Gesamtzusammensetzung der Komponente (A) und (iii) mindestens ein Urethandimethacrylat von 0,001 bis 20 Gew.-% in Bezug auf die Gesamtzusammensetzung der Komponente (A).

13. Verfahren zur Herstellung eines auspolymerisierten Prothesenbasismaterials, indem die Komponenten
A) mindestens eine flüssige Monomerkomponente, und
B) mindestens eine pulverförmige Komponente, des Prothesenmaterials nach einem der Ansprüche 1 bis 12, gemischt werden und nachfolgend auspolymerisiert werden.

14. Verfahren nach Anspruch 13, **dadurch gekennzeichnet, dass** die gemischten A) Monomerkomponente und die B) pulverförmige Komponente, insbesondere als auspolymerisierbares Prothesenbasismaterial in eine Negativform, wie eine Gussform, mindestens eines dentalen, prothetischen Formkörpers, wie Zahns, Aufbissschiene, Fräsrohlings, Dentalprothese, Teil von Prothese, Bohrschablone, Implantat, Mouthguard, Gelenkprothesen, Krone, Teleskop, Veneer, Zahnbrücke, Prothesenzahn, Implantateil, Abutment, Suprastruktur, kieferorthopädischer Apparat und Instrument, Hufteil eingeführt wird und, insbesondere unter erhöhtem Druck, insbesondere grösser gleich 2 bar, wie 2, 5 bis 10 bar, vorzugsweise 2 bis 4 bar, auspolymerisiert wird.

15. Auspolymerisiertes Prothesenmaterial erhältlich nach einem Verfahren nach einem der Ansprüche 13 oder 14.

16. Auspolymerisiertes Prothesenmaterial nach Anspruch 15, **dadurch gekennzeichnet, dass** es eine Bruchzähigkeit von ≥ 1,9 MPa · m^{1/2} und eine Gesamtbrucharbeit von ≥ 900 J/m² aufweist.

17. Verwendung von mit mindestens einer elastischen Phase modifizierten Kern-Schale Partikeln und mindestens einem Urethandimethacrylat in autopolymerisierbaren Prothesenmaterialien.

18. Kit umfassend ein autopolymerisierbares Prothesenmaterial, wobei das Kit separierte Komponenten (A) und (B) nach einem der Ansprüche 1 bis 12 umfasst.

## Claims

1. Autopolymerisable two-component prosthetic base material comprising
A) at least one liquid monomer component,
B) at least one powdered component,
**characterised in that**
the prosthetic material in component (A) comprises
(i) at least one initiator or one initiator system for autopolymerisation,
(ii) core-shell particles modified by an elastic phase, and
(iii) at least one urethane dimethacrylate.

2. Prosthetic base material according to claim 1, **characterised in that** the distribution of the elastic phase of the modified core-shell particles is selected from possibilities a to d
a) elastic phase as core in solid outer shell (core-shell-particles);
b) multiple elastic phases as cores in a solid matrix;
c) core-shell particles from a) distributed in solid matrix, and
d) solid core with elastic phase as outer shell.

3. Prosthetic base material according to claims 1 or 2, **characterised in that** the powdered component comprises
a) polymeric particles comprising polymers in the form of polymer powder comprising polyalkyl(meth)acrylates, optionally being crosslinked and being present as homopolymer or copolymer, whereby the polymers are based on at least one monomer comprising a (meth)acrylat group, selected from methylmethacrylate, ethylmethacrylate, propylmethacrylate, butylmethacrylate, n-hexylmethacrylate, 2-phenoxyethylmethacrylate, isobornylmethacrylate, isodecylmethacrylate, polypropylene glycol monomethacrylate, tetrahydrofurylmethacrylate, methylacrylate, ethylacrylate, propylacrylate, butylacrylate, n-hexylacrylate, 2-phenoxyethylacrylate, isobornylacrylate, isodecylacrylate, polypropylene glycol monoacrylate, tetrahydrofurylacrylate, hydroxyethylacrylate, hydroxypropylacrylate, hydroxyethylmethacrylate, hydroxypropylmethacrylate, a mixture comprising at least one of said (meth)acrylates and/or copolymers comprising one or at least two of the afore-mentioned monomers, polyamide particles, polyamide fibers, and optionally
b) inorganic fillers comprising pyrogenic or precipitated silicas, dental glasses, such as aluminosilicate glasses or fluoroaluminosilicate glasses, bariumaluminium silicate, strontium silicate, strontium borosilicate, lithium silicate, lithiumaluminium silicate, phyllosilicates, zeolites, amorphous spherical fillers based on oxide or mixed oxide, in particular mixed oxides of SiO₂ and ZrO₂, glass fibres and/or carbon fibers, as well as defined mixtures comprising said powdered components a) and b).

4. Prosthetic base material according to any one of the claims 1 to 3, **characterised in that** the powdered component comprises polymethylmethacrylate (PMMA) beads as polymeric particles and/or splitter polymerisates, copolymers, comprising as comonomers, being polymerised into the copolymer, styrene, alpha-methylstyrene, vinyltoluene, substituted vinyltoluene, such as vinylbenzylchloride, vinylhalogenide, such as vinylchloride, vinylester, such as vinylacetate, heterocyclic vinyl compounds, such as 2-vinylpyridine, and vinylpropionate, butadiene, isobutylene, 2-chlorobutadiene, 2-methylbutadiene, vinylpyridine, cyclopentene, (meth)acrylic acid ester, such as methylmethacrylate, butylmethacrylate, butylacrylate and hydroxyethylmethycrylate, further acrylonitrile, maleic acid and maleic acid derivatives, such as, for example, maleic acid anhydride, fumaric acid and fumaric acid derivatives, such as fumaric acid esters, acrylic acid, methacrylic acid, acryl(meth)acrylates, such as benzylmethacrylate or phenylmethacrylate, as well as, optionally, mixtures of said comonomers.

5. Prosthetic base material according to any one of the claims 1 to 4, **characterised in that** the elastic phase is selected from poly-(n-butyl acrylates) (PBA), butadiene-styrene copolymers, nitrile-butadiene copolymers, silicon rubber (graft copolymerisates), polyurethane polymerisates, polyolefin-based polyurethanes (polybutadiene-based polyurethanes), polydimethylsiloxane-modified polyurethanes, epoxy-functionalised elastic phases.

6. Prosthetic base material according to any one of the claims 1 to 5, **characterised in that** the solid shell, the solid core and/or the solid matrix are selected from (meth)acrylate polymers, preferably alkyl(meth)acrylate polymers, such as PMMA, polystyrene, epoxy-functionalised shell, metal oxide particles less than 100 nm as core, such as silicon dioxide, zirconium dioxide or silicon dioxide- and zirconium dioxide-containing particles, as well as homo-condensates or co-condensates of the afore-mentioned polymers.

7. Prosthetic base material according to any one of the claims 1 to 6, **characterised in that** the (iii) urethane dimethacrylate preferably is an urethane dimethacrylate, preferably selected from linear alkyl- or branched alkyl-functionalised urethane dimethacrylates, urethane dimethacrylate-functionalised polyethers, in particular bis(methacryloxy-2-ethoxycarbonylamino)alkylene, bis(methacryloxy-2-ethoxycarbonylamino)-substituted polyethers, preferably 1,6-bis(methacryloxy-2-ethoxycarbonylamino)-2,4,4-trimethylhexane.

8. Prosthetic base material according to any one of the claims 1 to 7, **characterised in that** the liquid monomer component comprises at least one monomer or a mixture of monomers from
a) methylmethacrylate, ethylmethacrylate, propylmethacrylate, butylmethacrylate, n-hexylmethacrylate, 2-phenoxyethylmethacrylate, isobornylmethacrylate, isodecylmethacrylate, polypropylene glycol monomethacrylate, tetrahydrofurylmethacrylate, methylacrylate, ethylacrylate, propylacrylate, butylacrylate, n-hexylacrylate, 2-phenoxyethylacrylate, isobornylacrylate, isodecylacrylate, polypropylene glycol monoacrylate, tetrahydrofurylacrylate, hydroxyethylacrylate, hydroxypropylacrylate, hydroxyethylmethacrylate, hydroxypropylmethacrylate, benzyl-, furfuryl- or phenyl(meth)acrylate, a mixture comprising at least one of said (meth)acrylates and/or copolymers comprising one or at least two of the afore-mentioned monomers, and/or
b) two- and/or multi-crosslinking agents comprising 1,4-butanediol dimethacrylate (1,4-BDMA) or pentaerythritol tetraacrylate, bis-GMA monomer (bisphenol-A-glycidylmethacrylate), triethylene glycol dimethacrylate (TEGDMA) and diethylene glycol dimethacrylate (DEGMA), tetraethylene glycol di(meth)acrylate, decanediol di(meth)acrylate, hexyldecanediol di(meth)acrylate, trimethylolpropane tri(meth)acrylate, pentaerythritol tetra(meth)acrylate, as well as butanediol di(meth)acrylate, ethylene glycol di(meth)acrylate, polyethylene glycol di(meth)acrylate, ethoxylated/propoxylated bisphenol-A-di(meth)acrylate, a mixture comprising at least one of said (meth)acrylates and/or copolymers comprising one or at least two of the afore-mentioned monomers.

9. Prosthetic base material according to any one of the claims 1 to 8, **characterised in that** the primary particles of the core-shell particles are from 500 nm to 10 nm.

10. Prosthetic base material according to any one of the claims 1 to 9, **characterised in** additionally comprising at least one initiator or at least one initiator system for autopolymerisation, which are present in the liquid component (A), in the powdered component (B) or in (A) and (B) depending on the reaction conditions and/or the polymerisation system.

11. Prosthetic base material according to any one of the claims 1 to 10, **characterised in that** the at least one initiator or the at least one initiator system for autopolymerisation comprises
a) at least one initiator, in particular at least one peroxide, perketal, perester, and/or one azo compound, and optionally
b) at least one activator, in particular at least one aromatic amine, or
c) at least one initiator system selected from redox systems, in particular a combination selected from dibenzoylperoxide, dilauroylperoxide and camphorquinone and amines selected from N,N-dimethyl-p-toluidine, N,N-dihydroxyethyl-p-toluidine, and p-dimethylamino-benzoic acid diethylester or a redox system comprising a peroxide, and a reduction agent selected from ascorbic acid, ascorbic acid derivative, barbituric acid or a barbituric acid derivative, sulfinic acid, sulfinic acid derivative, particularly preferred is a redox system comprising (i) barbituric acid or thiobarbituric acid or a barbituric acid derivative or thiobarbituric acid derivative, and (ii) at least one copper salt or one copper complex, and (iii) at least one compound having an ionic halogen atom, particularly preferred is a redox system comprising 1-benzyl-5-phenylbarbituric acid, copper acetylacetonate and benzyldibutylammoniumchloride.

12. Prosthetic base material according to any one of the claims 1 to 11, comprising
(ii) core-shell particles modified by at least one elastic phase of 0.001 to 20 % by weight, based on the total composition of component (A), and
(iii) at least one urethane dimethacrylate of 0.001 to 20 % by weight, based on the total composition of component (A).

13. Method for the production of a polymerised prosthetic base material, in which components
A) at least one liquid monomer component, and
B) at least one powdered component, of the prosthetic material according to any one of the claims 1 to 12, are being mixed and subsequently polymerised.

14. Method according to claim 13, **characterised in that** the
mixed A) monomer component and B) powdered component, in particular as being a polymerisable prosthetic base material, is being introduced into a negative form, such as a casting mould, of at least one dental prosthetic moulded body, such as tooth, occlusal splint, milling blank, dental prosthesis, part of prosthesis, surgical guide, implant, mouthguard, articular prostheses, crown, telescopic prosthesis and telescopic crown, veneer, dental bridge, prosthetic tooth, implant part, abutment, superstructure, orthodontic appliance and instrument, hoof part and being polymerised, in particular at elevated pressure, in particular greater than or equal to 2 bar, such as 2, 5 to 10 bar, preferably 2 to 4 bar.

15. Polymerised prosthetic base material obtainable by a method according to any one of the claims 13 or 14.

16. Polymerised prosthetic base material according to claim 15, **characterised in** having a fracture toughness of ≥ 1.9 Mpa · m^{1/2} and a total fracture work of ≥ 900 J/m².

17. Use of core-shell particles modified by at least one elastic phase and of at least one urethane (meth)acrylate in autopolymerisable prosthetic base materials.

18. Kit comprising an autopolymerisable prosthetic base material, whereby the Kit comprises separated components (A) and (B) according to any one of the claims 1 to 12.

## Revendications

1. Matériau de base prothétique à deux composantes autopolymérisable comprenant
A) au moins un composant monomère liquide,
B) au moins un composant pulvérulent,
**caractérisé en ce que**
le matériau prothétique dans le composant (A) comprend
(i) au moins un initiateur ou un système d'initiateurs pour l'autopolymerisation,
(ii) des particules coeur-coque modifiées par une phase élastique, et
(iii) au moins un diméthacrylate d'uréthane.

2. Matériau de base prothétique selon la revendication 1, **caractérisé en ce que** la distribution de la phase élastique des particules coeur-coque est sélectionnée à partir des possibilités a à d
a) une phase élastique en tant qu'un coeur dans une coque extérieure solide (des particules coeur-coque) ;
b) plusieurs phases élastiques en tant que des coeurs dans une matrice solide;
c) des particules coeur-coque de a) distribuées dans une matrice solide, et
d) un coeur solide avec une phase élastique en tant qu'une coque extérieure.

3. Matériau de base prothétique selon la revendication 1 ou 2, **caractérisé en ce que** le composant pulvérulent comprend
a) des particules polymériques comprenant des polymères sous forme d'une poudre polymère comprenant des poly(méth)acrylates d'alkyle, facultativement étant réticulés et étant présents en tant qu'un homopolymère ou copolymère, selon lequel les polymères reposent sur au moins un monomère comprenant un groupe (méth)acrylate, sélectionné à partir du méthacrylate de méthyle, méthacrylate d'éthyle, méthacrylate de propyle, méthacrylate de butyle, méthacrylate de n-hexyle, méthacrylate de 2-phenoxyethyle, méthacrylate d'isobornyle, méthacrylate d'isodecyle, monométhacrylate de polypropylène glycol, méthacrylate de tétrahydrofuryle, acrylate de méthyle, acrylate d'éthyle, acrylate de propyle, acrylate de butyle, acrylate de n-hexyle, acrylate de 2-phenoxyethyle, acrylate d'isobornyle, acrylate d'isodecyle, monoacrylate de polypropylène glycol, acrylate de tétrahydrofuryle, acrylate d'hydroxyéthyle, acrylate d'hydroxypropyle, méthacrylate d'hydroxyéthyle, méthacrylate d'hydroxypropyle, un mélange comprenant au moins une desdites (méth-)acrylates et/ou des copolymères comprenant un ou au moins deux des monomères susmentionnés, des particules de polyamide, des fibres de polyamide, et facultativement
b) des charges inorganique comprenant des silices pyrogéniques ou précipitées, des verres dentaires, comme des verres d'aluminosilicate ou des verres de fluoroaluminosilicate, baryumaluminiumsilicate, strontiumsilicate, strontiumborosilicate, lithiumsilicate, lithiumaluminiumsilicate, des phyllosilicates, des zéolites, des charges amorphes sphériques sur la base d'oxides ou d'oxide mixte, en particulière des oxides mixtes de SiO₂ et ZrO₂, des fibre de verre et/ou des fibres de carbone, ainsi que des mélanges défini comprenant lesdites composants pulvérulents a) et b).

4. Matériau de base prothétique selon l'une des revendications 1 à 3, **caractérisé en ce que** le composant pulvérulent comprend des perles du polyméthacrylate de méthyle (PMMA) en tant que des particules polymériques et/ou des polymérisats en éclats, des copolymères, comprenant en tant que des monomères, étant polymérisé dans le copolymère, le styrène, l'alpha-méthylstyrène, le vinyltoluène, des vinyltoluènes substitués, comme le chlorure de vinylbenzyle, l'halogénures de vinyle, comme le chlorure de benzyle, des vinylesters, comme l'acétate de vinyle, des composés vinyliques hétérocycliques, comme le 2-vinylpyridine, l'acétate de vinyle et le propionate de vinyle, le butadiène, l'isobutylène, le 2-chlorobutadiène, le 2-méthylbutadiène, le vinylpyridine, le cyclopentène, des esters d'acide (meth)acrylique, comme le méthacrylate de méthyle, le méthacrylate de butyle, l'acrylate de butyle et le méthacrylate d'hydroxyéthyle, en outre l'acrylonitrile, l'acide maléique et des dérivés d'acide maléique, comme pour example l'anhydride d'acide maléique, l'acide fumarique ou des dérivés d'acide fumarique, comme l'ester d'acide fumarique, l'acide acrylique, l'acide méthacrylique, des (méth)acrylates d'aryle, comme le méthacrylate de benzyle ou le méthacrylate de phényle, ainsi que, facultativement, des mélanges desdites comonomères.

5. Matériau de base prothétique selon l'une des revendications 1 à 4, **caractérisé en ce que** la phase élastique est sélectionnée à partir des poly-(n-butyle acrylates) (PBA), des copolymères de butadiène-styrène, des copolymères de nitrile-butadiène, des (copolymérisats greffés) de caoutchouc silicone, des polymérisats de polyuréthane, des polyuréthanes sur la base de la polyoléfine (des polyuréthanes sur la base du polybutadiène), des polyuréthanes modifiés par le polydiméthylsiloxane, des phases élastiques fonctionnalisées par l'époxy.

6. Matériau de base prothétique selon l'une des revendications 1 à 5, **caractérisé en ce que** la coque solide, le coeur solide et/ou la matrice solide sont sélectionnés à partir des polymères de (méth)acrylate, de préférence des polymères de (méth)acrylate d'alkyle, comme PMMA, polystyrène, une coque fonctionnalisée par l'époxy, des particules d'oxide métallique inférieur de 100 nm en tant qu'un coeur, comme le dioxyde de silicium, le dioxyde de zirconium ou des particules contenant le dioxyde de silicium et le dioxyde de zirconium, ainsi que des homo-condensats ou co-condensats des polymères susmentionnés.

7. Matériau de base prothétique selon l'une des revendications 1 à 6, **caractérisé en ce que** le (ii) diméthacrylate d'uréthane de préférence est sélectionné à partir des diméthacrylates d'uréthane fonctionnalisés par l'alkyl linéaire ou ramifié, des polyéthers fonctionnalisés par diméthacrylate d'uréthane, en particulière le bis(méthacryloxy-2-éthoxycarbonylamino)alkylène, des polyéthers substitués par bis(méthacryloxy-2-éthoxycarbonylamino), de préférence 1,6-bis(méthacryloxy-2-éthoxycarbonylamino)-2,4,4-triméthylhexane.

8. Matériau de base prothétique selon l'une des revendications 1 à 7, **caractérisé en ce que** le composant monomère liquide comprend au moins une monomère ou un mélange de monomères à partir
a) du méthacrylate de méthyle, méthacrylate d'éthyle, méthacrylate de propyle, méthacrylate de butyle, méthacrylate de n-hexyle, méthacrylate de 2-phenoxyethyle, méthacrylate d'isobornyle, méthacrylate d'isodecyle, monométhacrylate de polypropylène glycol, méthacrylate de tétrahydrofuryle, de l'acrylate de méthyle, l'acrylate d'éthyle, l'acrylate de propyle, l'acrylate de butyle, l'acrylate de n-hexyle, l'acrylate de 2-phenoxyethyle, l'acrylate d'isobornyle, l'acrylate d'isodecyle, le monoacrylate de polypropylène glycol, l'acrylate de tétrahydrofuryle, l'acrylate d'hydroxyéthyle, l'acrylate d'hydroxypropyle, du méthacrylate d'hydroxyéthyle, du méthacrylate d'hydroxypropyle, du (méth)acrylate de benzyle, furfuryle ou phényle, d'un mélange comprenant au moins une desdites (méth-)acrylates et/ou des copolymères comprenant un ou au moins deux des monomères susmentionnés, et/ou
b) des agents de réticulations double et/ou multiple comprenant le diméthacrylate de 1,4-butanediol (1,4-BDMA) ou le tétraacrylate de pentaérythritol, le monomére bis-GMA (bisphenol-A-glycidylméthacrylate), le diméthacrylate de triéthylène glycol (TEGDMA) et le diméthacrylate de diéthylène glycol (DEGMA), le di(méth)acrylate de tétraéthylène glycol, le di(méth)acrylate de decanediol, le di(méth)acrylate, d'hexyldecanediol, le tri(méth)acrylate de triméthylolpropane, le tetra(méth)acrylate de pentaérythritol, ainsi que le di(méth)acrylate de butanediol, le di(méth)acrylate d'ethylène glycol, le di(méth)acrylate de polyéthylène glycol, le bisphenol-A-di(méth)acrylate éthoxylé/propoxylé, un mélange comprenant au moins une desdites (méth-)acrylates et/ou des copolymères comprenant un ou au moins deux des monomères susmentionnés.

9. Matériau de base prothétique selon l'une des revendications 1 à 8, **caractérisé en ce que** les particules primaires des particules coeur-coque sont de 500 nm à 10 nm.

10. Matériau de base prothétique selon l'une des revendications 1 à 10, caractérisé en additionnellement comprenant au moins un initiateur ou un système d'initiateurs pour l'autopolymerisation, qui sont présents dans le composant liquide (A), dans la composant pulvérulent (B) ou dans (A) et (B), au gré des conditions de réaction et/ou le système de polymérisation.

11. Matériau de base prothétique selon l'une des revendications 1 à 10, **caractérisé en ce que** l'au moins un initiateur ou le système d'initiateurs pour l'autopolymerisation comprend
a) au moins un initiateur, en particulière au moins un peroxyde, un percétal, un perester et/ou un composé azoïque, et facultativement
b) au moins un activateur, en particulière une amine aromatique, ou
c) au moins un système d'initiateurs sélectionné à partir des systèmes redox, en particulière une combinaison sélectionnée à partir du peroxyde de benzoyle, dilauoryle et du camphoquinone avec des amines sélectionnées du N,N-diméthyle-p-toluidine, N,N-dihydroxyéthyle-p-toluidine, et du diethylester d'acide p-diméthylamino benzoïque ou un système redox comprenant un peroxyde et un agent réducteur sélectionné à partir de l'acide ascorbique, un dérivé d'acide ascorbique, l'acide barbiturique ou un dérivé d'acide barbiturique, l'acide sulfinique, un dérivé d'acide sulfinique, particulièrement préféré est un système redox comprenant (i) l'acide barbiturique ou l'acide thiobarbiturique ou un dérivé d'acide barbiturique ou d'acide thiobarbiturique, et (ii) au moins au moins un sel de cuivre ou un complexe de cuivre, et (iii) au moins un composé ayant un atome d'halogène ionique, particulièrement préféré est un système redox comprenant l'acide de 1-benzyle-5-phényle barbiturique, l'acétylacétonate de cuivre et le chlorure de benzyldibutylammonium.

12. Matériau de base prothétique selon l'une des revendications 1 à 11, comprenant
(ii) des particules coeur-coque modifiées par au moins une phase élastique de 0,0001 à 20 % en poids, sur la base de la composition totale du composant (A), et
(iii) au moins un diméthacrylate d'uréthane de 0,0001 à 20 % en poids, sur la base de la composition totale de composant (A).

13. Procédé pour la production d'un matériau de base prothétique polymérisé, dans lequel les composants
A) au moins un composant monomère liquide, et
B) au moins un composant pulvérulent, du matériau prothétique selon l'une des revendications 1 à 12, sont étant mélangés et subséquemment polymérisés.

14. Procédé selon la Revendications 13, **caractérisé en ce que**
le A) composante monomère et le B) composant pulvérulent mélangés, en particulière en tant qu'un matériau de base prothétique polymérisable, est étant introduit dans une forme négatif, comme un moule, d'au moins un corps moulé dentaire prothétique, comme une dent, une gouttière occlusale, un ébauche de fraisage, une prothèse dentaire, un part de la prothèse, un guide chirurgical, un implant, un protège-dents, des prothèses articulaires, une couronne, une prothèse télescopique et une couronne télescopique, une facette, un bridge dentaire, un dent prothétique, un part d'implant, un aboutement, une superstructure, un appareil et un instrument orthodontique, un part de sabot, et étant polymérisé, en particulière à pression plus élevée, en particulière égal ou supérieur à 2 bar, comme 2, 5 à 10 bar, de préférence 2 à 4 bar.

15. Matériau de base prothétique polymérisé obtenable par un procédé selon l'une des revendications 13 ou 14.

16. Matériau de base prothétique polymérisé selon la revendication 15, caractérisé en ayant une ténacité à la rupture de ≥ 1.9 Mpa · m^{1/2} et une énergie à la rupture totale de ≥ 900 J/m².

17. Utilisation des particules coeur-coque modifiées par au moins une phase élastique et au moins un diméthacrylate d'uréthane dans des matériaux de base prothétiques autopolymérisables.

18. Kit comprenant un matériau de base prothétique autopolymérisable, selon lequel le kit comprend des composantes (A) et (B) séparés selon l'une des Revendications 1 à 12.
